# EUROPÄISCHE PATENTANMELDUNG

(11) **EP 2 113 498 A1**
(43) Veröffentlichungstag der Anmeldung: **04.11.2009**
(21) Anmeldenummer: 09158738.6
(22) Anmeldetag: 24.04.2009
(51) Int. Cl.: C07C 253/28, B01J 23/00, C07C 51/255

(54) **Verfahren zur Partialoxidation oder Ammoxidation mit einem Eisenmolybdat-Katalysator**

(30) Priorität: 25.04.2008 EP 08155228
(71) Anmelder: BASF SE, 67056 Ludwigshafen (DE)
(72) Erfinder: Horstmann, Catharina, 67056 Ludwigshafen (DE); Altwasser, Stefan, 67157 Wachenheim (DE); Rosowski, Frank, 68239 Mannheim (DE)
(74) Vertreter: Reitstötter - Kinzebach

(57) **Zusammenfassung**

Beschrieben wird ein Verfahren zur Partialoxidation oder Ammoxidation von aromatischen Kohlenwasserstoffen in der Gasphase unter Verwendung eines Katalysators, dessen katalytisch aktive Masse ein Metallmischoxid der allgemeinen Formel I enthält

Mo₃Fe_{b}XₓO_{z}, I

worin X für wenigstens ein unter Ag, Te, K, Bi, Au, Ce, Co, Cs, Cu, Eu, Mn, Rb, Sb, Se, Si, V, W ausgewähltes Metall steht, b einen Wert von 1,6 bis 2,5 hat, x einen Wert von 0,01 bis 3,0 hat und z eine Zahl, die sich durch die Wertigkeit und Häufigkeit der von Sauerstoff verschiedenen Elemente in der Formel I bestimmt, bedeutet. Die Katalysatoren sind hoch selektiv.

## Beschreibung

Die Erfindung betrifft ein Verfahren zur Partialoxidation oder Ammoxidation von aromatischen Kohlenwasserstoffen in der Gasphase.

Eine Vielzahl von Aldehyden, Carbonsäuren und/oder Carbonsäureanhydriden wird technisch durch die katalytische Gasphasenoxidation von aromatischen Kohlenwasserstoffen wie Benzol, o-, m- oder p-Xylol, Naphthalin, Toluol oder Durol (1,2,4,5-Tetramethylbenzol) in Festbettreaktoren hergestellt. Dabei werden je nach Ausgangsmaterial beispielsweise Benzaldehyd, Benzoesäure, Maleinsäureanhydrid, Phthalsäureanhydrid, Isophthalsäure, Terephthalsäure oder Pyromellithsäureanhydrid gewonnen. Dazu wird im Allgemeinen ein Gemisch aus einem molekularen Sauerstoff enthaltenden Gas, beispielsweise Luft und das zu oxidierende Ausgangsmaterial durch Rohre geleitet, in denen sich eine Schüttung eines Katalysators befindet.

Im großtechnischen Maßstab werden aromatische Nitrile vielfach durch Ammoxidation (Ammonoxidation) hergestellt. Die Ammoxidation ist ein heterogen-katalytischer Prozess, bei dem alkylsubstituierte Aromaten durch Umsetzung mit Ammoniak und Sauerstoff in Gegenwart von Übergangsmetall-Katalysatoren in Nitrile umgewandelt werden. So ist z. B. o-Phthalodinitril, welches für die Pigmentherstellung große technische Bedeutung hat, durch Ammonoxidation von o-Xylol erhältlich.

Wenxing Kuang, Yining Fan und Yi Chen beschreiben in Langmuir 2000, 16, 5205-5208 die Herstellung ultrafeiner Eisen-Molybdän-Oxide. Diese werden als Katalysator zur Partialoxidation von Toluol getestet.

Die FR 2465714 offenbart ein Verfahren zur Herstellung von Phthalonitrilen durch Ammoxidation von Xylol in Gegenwart eines Katalysators, der Oxide von Antimon, Bismuth, Vanadium oder Molybdän und Eisen enthält.

Die DE-OS 2 136 779 offenbart ein Verfahren zur Herstellung von Benzaldehyd durch katalytische Oxidation von Toluol in der Gasphase in Gegenwart eines sauerstoffhaltigen Katalysators, der Molybdän und wenigstens ein unter Fe, Ni, Co, Sb, Bi, V, P, Sm, Ta, Sn, Cr enthält.

Die DE-OS 2 314 151 beschreibt ein Verfahren zur Herstellung eines aromatischen Nitrils oder eines heterocyclischen aromatischen Nitrils durch Umsetzung Methylsubstituierter aromatischer Verbindungen mit Sauerstoff und Ammoniak in Gegenwart eines Katalysators der Zusammensetzung FeₐBi_{b}Mo_{c}Ni_{d}CoₑQ_{f}R_{g}Oₓ, worin Q für ein Alkalimetall, Erdalkalimetall oder Thallium; R für B, P, As, Sb oder W steht; a und b für 0,1 bis 12; c für 8 bis 16; d, e und f für 0 bis 10; g für 0 bis 6 steht und x so gewählt ist, dass die freien Valenzen der anderen Elemente abgebunden sind.

Die EP-A 92 677 beschreibt ein Verfahren zur Herstellung substituierter Benzaldehyde durch Oxidation eines entsprechenden Methylbenzols in Anwesenheit eines Katalysators der Zusammensetzung MoMe¹ₐMe²_{b}Oₓ, worin Me¹ für Cu oder Ag und Me² für Ti, Zr, Fe, Co, Ni, Zn, Sn, Pb, Sb, Bi, B, P und/oder ein Seltenerdmetall steht, a für 0,2 bis 1,0, b für 0 bis 0,5 steht und x für die Anzahl der zum Ansättigen der Valenzen der anderen Elemente erforderlichen Sauerstoffatome steht.

Die EP-A beschreibt ein Verfahren zur Herstellung von p-substituierten Benzaldehyden durch Dampfphasenoxidation eines entsprechenden Alkylbenzols in gegenwart eines Metalloxidkatalysators, der neben Molybdän die Elemente Bi, Co, Ni, Fe, W, Nb, Ta, K, Rb, Cs, TI, In, P, B, Sb, V, Sr, Cr, Mn, Re, Pd, Ir, Rh, Cu, Sn, Zn, Sm, Mg, Ce, Ag, Li, As, Ba und Ca enthalten kann. Im Bespiel 17 dieser Druckschrift wird ein Katalysator der Zusammensetzung Mo₁₂Fe₆K_{0,06}Oₓ verwendet; dabei wird eine Selektivität von 29% erzielt.

Die WO 2007/042369 beschreibt ein Verfahren zur Herstellung von Aldehyden und Säuren durch Oxidation von Olefinen oder methylierten Aromaten mit Luft oder Sauerstoff, wobei man einen Katalysator der allgemeinen Formel (Mo₁₂ Biₐ C_{b}(Co+Ni)_{c} D_{d} Eₑ F_{f} Gg Hₕ) Oₓ einsetzt,
in der bedeuten C: Fe, D: W, P; E: Li, K, Na, Rb, Cs, Mg, Ca, Ba, Sr; F: Ce, Mn, Cr, V; G: Nb, Se, Te, Sm, Gd, La, Y, Pd, Pt, Ru, Ag, Au; H: Si, Al, Ti, Zr; und a = 0 - 5,0; b = 0,5 - 5,0; c = 2 - 15; d = 0,01 - 5,0; e = 0,001 - 2; f = 0, 001 - 5; g = 0 - 1,5; h = 0 - 800; und x = Zahl, die von der Wertigkeit und Häufigkeit der von Sauerstoff verschiedenen Elemente bestimmt wird.

Die NL 7209921 beschreibt eine Gasphasenoxidation von Toluol zu Benzaldehyd bei 200 - 600°C an einem Katalysator, der Molybdän und ein weiteres Element M (M = Fe, Ni, Co, Sb, Bi, V, P, Sm, Ta, Sm, Cr) im Verhältnis M/Mo < 1 enthält.

Der Erfindung liegt die Aufgabe zu Grunde, neue hoch selektive Katalysatoren und Verfahren zur Partialoxidation oder Ammoxidation von aromatischen Kohlenwasserstoffen in der Gasphase bereit zu stellen.

Die Aufgabe wird gelöst durch ein Verfahren zur Partialoxidation oder Ammoxidation von aromatischen Kohlenwasserstoffen in der Gasphase unter Verwendung eines Katalysators, dessen katalytisch aktive Masse ein Metallmischoxid der allgemeinen Formel I enthält

Mo₃Fe_{b}XₓO_{z}, I

worin
- X: für wenigstens ein unter Ag, Te, K, Bi, Au, Ce, Co, Cs, Cu, Eu, Mn, Rb, Sb, Se, Si, V, W ausgewähltes Metall steht,
- b: einen Wert von 1,6 bis 2,5 hat,
- x: einen Wert von 0,01 bis 3,0 hat
- z: eine Zahl, die sich durch die Wertigkeit und Häufigkeit der von Sauerstoff ver- schiedenen Elemente in der Formel I bestimmt, bedeutet.

Die Erfindung betrifft außerdem einen Gasphasenoxidations-Katalysator, dessen katalytisch aktive Masse das vorstehend definierte Metallmischoxid der allgemeinen Formel I umfasst.

Die folgenden Ausführungen mit Bezug auf das erfindungsgemäße Verfahren gelten entsprechend für den erfindungsgemäßen Katalysator und umgekehrt, soweit aus dem Kontext nicht anders ersichtlich.

In bevorzugten Ausführungsformen hat b einen Wert von 1,8 bis 2,3, insbesondere 1,9 bis 2,1.

In bevorzugten Ausführungsformen hat x einen Wert von 0,05 bis 1, insbesondere 0,4 bis 0,6.

In bevorzugten Ausführungsformen steht X für wenigstens ein unter Ag, Te, K und Bi ausgewähltes Metall, insbesondere für Ag.

Die Phase des Metallmischoxids besteht vorzugsweise im Wesentlichen aus einer Zusammensetzung der Formel I, in der weitere, von der Formel I nicht erfasste Elemente abwesend sind.

Der eingesetzte aromatische Kohlenwasserstoff ist eine Verbindung mit wenigstens einer carbocylischen oder heterocylischen aromatischen Ringstruktur, die unter den Bedingungen einer Gasphasenpartialoxidation zu Aldehyden, Carbonsäuren und/oder Carbonsäureanhydriden umgesetzt werden kann oder unter den Bedingungen einer Ammoxidation in der Gasphase zu Nitrilen umgesetzt werden kann. Es eignen sich insbesondere ein- oder mehrfach alkylierte Aromaten, insbesondere methylierte und/oder ethylierte Aromaten, und kondensierte Aromaten.

Die aromatischen Stammverbindungen können Substituenten tragen, welche sich unter den Bedingungen der Partialoxidation oder Ammoxidation inert verhalten, also z.B. Halogen oder die Trifluormethyl-, Nitro-, Amino- oder Cyano-Gruppe. Auch nicht-inerte Substituenten kommen in Betracht, wenn sie unter Bedingungen der Partialoxidation oder Ammonoxidation in erwünschte Substituenten übergehen, wie beispielsweise die Aminomethyl- oder die Hydroxymethylgruppe.

Bevorzugte aromatische Kohlenwasserstoffe sind Toluol, o-Xylol, m-Xylol, p-Xylol und/oder Naphthalin sowie Methylpyridine.

Eine Ausführungsform des erfindungsgemäßen Verfahrens zur Partialoxidation betrifft die Herstellung von m-Tolylaldehyd aus m-Xylol.

Eine Ausführungsform des erfindungsgemäßen Verfahrens zur Partialoxidation betrifft die Herstellung von o-Toluylaldehyd und/oder Phthalsäureanhydrid aus o-Xylol und/oder Naphthalin.

Weitere Ausführungsformen des erfindungsgemäßen Verfahrens zur Partialoxidation betreffen die Herstellung von Benzoesäure und/oder Benzaldehyd aus Toluol zu, oder von Pyridincarbonsäuren, wie Nicotinsäure, aus Methylpyridinen, wie β-Picolin.

Ausführungsformen des erfindungsgemäßen Verfahrens zur Ammoxidation betreffen die Herstellung von o-Phthalodinitril aus o-Xylol, von Isophthalodinitril aus m-Xylol, von Terephthalodinitril aus p-Xylol, von Benzonitril aus Toluol und von Nicotinsäurenitril aus β-Picolin.

Die erfindungsgemäßen Metallmischoxide sind auf verschiedene Weise erhältlich. Sie können z. B. durch eines der folgenden Verfahren hergestellt werden:
(i) Umsetzung wenigstens einer Molybdänquelle, einer Eisenquelle und einer Quelle des Metalls X;
(ii) Behandeln von Eisenmolybdat, z. B. der Formel (Fe₂(MoO₄)₃), mit einer Quelle des Metalls X.

Anschließend werden die so erhaltenen Massen in der Regel calciniert.

Zur Herstellung des Metallmischoxids gemäß (i) werden im Allgemeinen eine Molybdänquelle, eine Eisenquelle und eine Quelle des Metalls X innig miteinander vermengt. Das Vermengen erfolgt vorzugsweise in einer Lösung und/oder Suspension in einem Lösungsmittel. Als Lösungsmittel können polare organische Lösungsmittel, wie Polyole, Polyether oder Amine, z. B. Pyridin, dienen, bevorzugt wird als Lösungsmittel Wasser verwendet.

Als Molybdänquelle, Eisenquelle und Quelle des Metalls X werden Oxide oder Verbindungen der Elemente verwendet, die beim Erwärmen, zumindest beim Erwärmen in Anwesenheit von Sauerstoff, z. B. an der Luft, in Oxide überführbar sind. Hierzu zählen Hydroxide, Oxid-hydroxide, Carboxylate, Carbonate, Perchlorate, Halogenide und insbesondere Nitrate.

Als Molybdänquelle wird bevorzugt Ammoniumheptamolybdat verwendet, die Verwendung anderer Molybdänverbindungen, z. B. Molybdän(VI)-oxid, ist ebenfalls möglich.

Als Eisenquelle ist Eisen(II)-nitrat bevorzugt. die Verwendung anderer Eisenverbindungen, z. B. Eisen(II)-acetat, Eisen(III)-citrat, Eisen(II)-oxalat, Eisen(II)-chlorid, ist ebenfalls möglich.

Als Quelle der Metallkomponente X werden in der Regel solche Verbindungen gewählt, die im verwendeten Lösungsmittel löslich sind. Wird Wasser als Lösungsmittel bei der Herstellung der Metallmischoxide verwendet, können beispielsweise die Carboxylate, insbesondere die Acetate, der Metallkomponente X eingesetzt werden. Bevorzugt werden die Nitrate der betreffenden Metallkomponente X verwendet.

Je nach der gewünschten chemischen Zusammensetzung des Metallmischoxids der Formel I werden zu dessen Herstellung die sich aus b und x von Formel I ergebenden Mengen von Molybdänquelle, Eisenquelle und Quelle des Metalls X vermengt.

Das Vermengen der Molybdänquelle, Eisenquelle und Quelle des Metalls X kann im Allgemeinen bei Raumtemperatur oder bei erhöhter Temperatur durchgeführt werden. In der Regel wird die Umsetzung bei Temperaturen von 5 bis 375 °C, vorzugsweise bei 10 bis 100 °C und besonders bevorzugt bei 15 bis 30 °C vorgenommen. Liegt die Temperatur der Umsetzung oberhalb der Temperatur des Siedepunktes des verwendeten Lösungsmittels, wird die Umsetzung zweckmäßigerweise unter dem Eigendruck des Reaktionssystems in einem Druckgefäß ausgeführt. Vorzugsweise werden die Reaktionsbedingungen so gewählt, dass die Umsetzung bei Atmosphärendruck durchgeführt werden kann. Die Dauer dieser Umsetzung kann in Abhängigkeit von der Art der umgesetzten Ausgangsmaterialien und den angewandten Temperaturbedingungen wenige Minuten bis mehrere Tage betragen.

Das so gebildete Gemenge kann aus der Reaktionsmischung isoliert und bis zur weiteren Verwendung gelagert werden. Die Isolierung kann z. B. bevorzugt durch Entfernen des Lösungsmittels und Trocknen des erhaltenen Feststoffs erfolgen, wobei die Trocknung sowohl in herkömmlichen Trocknern, wie Walzentrockner, aber auch z. B. in Gefriertrocknern durchgeführt werden kann. Ebenfalls kann die Trocknung der erhaltenen Lösung und/oder Suspension mittels Sprühtrocknung durchgeführt werden. Die Sprühtrocknung wird im Allgemeinen unter Atmosphärendruck oder vermindertem Druck vorgenommen. Je nach angewandtem Druck und verwendetem Lösungsmittel bestimmt sich die Eingangstemperatur des Trocknungsgases. Im Allgemeinen wird als Trocknungsgas Luft verwendet, es können aber selbstverständlich auch andere Trocknungsgase wie Stickstoff oder Argon, benutzt werden. Die Eingangstemperatur des Trocknungsgases in den Sprühtrockner wird vorteilhaft so gewählt, dass die Ausgangstemperatur des durch Verdampfung des Lösungsmittels abgekühlten Trocknungsgases 200 °C für einen längeren Zeitraum nicht übersteigt. In der Regel wird die Ausgangstemperatur des Trocknungsgases auf 50 bis 150 °C, vorzugsweise 100 bis 140 °C eingestellt.

Bei der Herstellungsart (ii) behandelt man Eisenmolybdat mit einer Quelle des Metalls X. Hierzu kann man pulverförmiges oder kompaktiertes Eisenmolybdat mit einer Lösung, z. B. einer wässrigen Lösung, einer Verbindung von X tränken und dann trocknen. Zweckmäßigerweise kann man vorab das Wasseraufnahmevermögen des Eisenmolybdats bestimmen und dann mit einem Lösungsvolumen tränken, das dem zuvor ermittelten Aufnahmevermögen entspricht. Das Eisenmolybdat kann man beispielsweise erhalten, indem man in eine Lösung von Ammoniumheptamolybdat langsam unter Rühren eine Eisennitratlösung gibt und das Gemisch anschließend trocknet.

Man kann auch pulverförmiges Eisenmolybdat, z. B. getrocknetes, pulverförmiges Eisenmolybdat-Gel in einer Lösung oder Suspension einer Verbindung von X einbringen und dann das Lösungsmittel entfernen.

Zum Calcinieren wird das Gemenge auf eine Temperatur von wenigstens 300 °C, insbesondere 400 bis 500 erwärmt. Das Calcinieren erfolgt üblicherweise in Gegenwart von molekularem Sauerstoff, vorzugsweise an der Luft. Das Calcinieren kann auch erfolgen, nachdem die Masse auf inerte Träger aufgebracht worden ist, wie nachstehend beschrieben.

Das Metallmischoxid kann in Form eines Vollkatalysators oder vorzugsweise in Form eines Schalenkatalysators für die Partialoxidation oder Ammoxidation in der Gasphase eingesetzt werden.

Zur Herstellung eines Vollkatalysators wird die pulverförmiger Metallmischoxidmasse z. B. durch z. B. durch Tablettieren, Extrudieren oder Strangpressen von zur gewünschten Katalysatorgeometrie verdichtet. Zur Vollkatalysatorherstellung können neben der pulverförmigen Metallmischoxidmasse gegebenenfalls Hilfsmittel wie z. B. Graphit oder Stearinsäure als Gleitmittel und/oder Formhilfsmittel und Verstärkungsmittel wie Mikrofasern aus Glas, Asbest, Siliciumcarbid oder Kaliumtitanat mitverwendet werden.

Zur Herstellung von Schalenkatalysatoren wird die pulverförmiger Metallmischoxidmasse auf vorgeformte inerte Katalysatorträger geeigneter Geometrie aufgebracht.

Als inertes Trägermaterial können praktisch alle Trägermaterialien des Standes der Technik, wie sie vorteilhaft bei der Herstellung von Schalenkatalysatoren eingesetzt werden, Verwendung finden, beispielsweise Quarz (SiO₂), Porzellan, Magnesiumoxid, Zinndioxid, Siliciumcarbid, Rutil, Tonerde (Al₂O₃), Aluminiumsilikat, Steatit (Magnesiumsilikat), Zirkoniumsilikat, Cersilikat oder Mischungen dieser Trägermaterialien. Als vorteilhafte Trägermaterialien sind insbesondere Steatit und Siliciumcarbid hervorzuheben.

Das Trägermaterial ist in der Regel nicht-porös. Der Ausdruck "nicht-porös" ist dabei im Sinne von "bis auf technisch unwirksame Mengen an Poren nicht-porös" zu verstehen, da technisch unvermeidlich eine geringe Anzahl Poren im Trägermaterial, das idealerweise keine Poren enthalten sollte, vorhanden sein können.

Die Form des Trägermaterials ist für die erfindungsgemäßen Schalenkatalysatoren im Allgemeinen nicht kritisch. Beispielsweise können Katalysatorträger in Form von Kugeln, Ringen, Tabletten, Spiralen, Röhren, Extrudaten oder Splitt verwendet werden. Die Dimensionen dieser Katalysatorträger entsprechen denen üblicherweise zur Herstellung von Schalenkatalysatoren für die Gasphasenpartialoxidation von aromatischen Kohlenwasserstoffen verwendeten Katalysatorträgern.

Zur schalenförmigen Beschichtung des inerten Trägermaterials werden bekannte Verfahren angewendet. Beispielsweise kann die pulverförmige Metallmischoxidmasse oder ein pulverförmiger Vorläufer, aber auch eine Suspension der Aktivmasse oder eines Vorläufers in einer Dragiertrommel gegebenenfalls bei erhöhter Temperatur auf den Katalysatorträger aufgebracht werden. Anstelle von Dragiertrommeln können auch Wirbelbettbeschichter eingesetzt werden.

Der Haftvermittler bzw. das Suspensionsmedium ist im Allgemeinen Wasser, dem vorzugsweise Bindemittel wie höhere Alkohole, mehrwertige Alkohole, z. B. Ethylenglykol, 1,4-Butandiol oder Glycerin, Dimethylformamid, Dimethylacetamid, Dimethylsulfoxid, N-Methylpyrrolidon oder cyclische Harnstoffe, wie N,N'-Dimethylethylenharnstoff oder N,N'-Dimethylpropylenharnstoff, oder (Co)Polymere, gelöst oder vorteilhaft in Form einer wässrigen Dispersion zugesetzt werden, wobei im Allgemeinen Bindemittelgehalte von 10 bis 20 Gew.- %, bezogen auf den Feststoffgehalt der Suspension geeignet sind. Geeignete polymere Bindemittel sind z. B. Vinylacetat/Vinyllaurat-, Vinylacetat/Acrylat-, Styrol/Acrylat-, Vinylacetat/Maleat- oder Vinylacetat/Ethylen-Copolymere. Bei einer thermischen Behandlung bei Temperaturen über 200 bis 500 °C entweicht das Bindemittel durch thermische Zersetzung und/oder Verbrennung aus der aufgetragenen Schicht.

Die Schichtdicke der Katalysatorschale bzw. die Summe der Schichtdicken der Schalen, die die katalytisch aktiven Bestandteile enthalten, beträgt im Allgemeinen 10 bis 250 µm. Der Anteil des Metallmischoxids der Formel I am fertigen Katalysator beträgt in der Regel 5 bis 35 Gew.-%, vorzugsweise 10 bis 30 Gew.-%.

Die erfindungsgemäßen Katalysatoren werden für die Partialoxidation von aromatischen Kohlenwasserstoffen zu Aldehyden, Carbonsäuren und/oder Carbonsäureanhydriden, mit einem molekularen Sauerstoff enthaltenden Gas verwendet. Hierzu bringt man ein gasförmiges Gemisch, das den aromatischen Kohlenwasserstoff und molekularen Sauerstoff enthält, bei erhöhter Temperatur, z. B. 100 bis 650 °C und vorzugsweise 300 bis 550 °C, mit dem Katalysator in Kontakt. Die Umsetzung kann in einem Festbettreaktor mit ortfest angeordnetem Katalysatorfestbett oder einem Wirbelschichtreaktor erfolgen.

Vorzugsweise erfolgt die Umsetzung in einem Festbettreaktor. Die erfindungsgemäßen Katalysatoren werden hierzu in die Reaktionsrohre eines Röhrenreaktors gefüllt, die von außen, z. B. mittels einer Salzschmelze, auf die Reaktionstemperatur thermostatisiert werden. Über die so bereitete Katalysatorschüttung wird das Reaktionsgas bei Temperaturen von 100 bis 650 °C und vorzugsweise 300 bis 550 °C und bei einem Überdruck von im Allgemeinen 0,1 bis 2,5 bar, vorzugsweise von 0,2 bis 0,5 bar mit einer Raumgeschwindigkeit von im Allgemeinen 750 bis 5000 h^{- 1} geleitet.

Das dem Katalysator zugeführte Reaktionsgas wird im Allgemeinen durch Vermischen von einem molekularen Sauerstoff enthaltenden Gas, das außer Sauerstoff noch geeignete Reaktionsmoderatoren und/oder Verdünnungsmittel, wie Wasserdampf, Kohlendioxid und/oder Stickstoff, enthalten kann, mit dem zu oxidierenden, aromatischen Kohlenwasserstoff erzeugt, wobei das molekularen Sauerstoff enthaltende Gas im Allgemeinen 1 bis 100 Vol.-%, vorzugsweise 2 bis 50 Vol.-% und besonders bevorzugt 10 bis 30 Vol.-% Sauerstoff, 0 bis 30 Vol.-%, vorzugsweise 0 bis 20 Vol.-% Wasserdampf sowie 0 bis 50 Vol.-%, vorzugsweise 0 bis 1 Vol.-% Kohlendioxid, Rest Stickstoff, enthalten kann. Zur Erzeugung des Reaktionsgases wird das molekularen Sauerstoff enthaltende Gas im Allgemeinen mit 30 bis 300 g je Nm³, bevorzugt mit 70 bis 150 g je Nm³ Gas des zu oxidierenden aromatischen Kohlenwasserstoffs beschickt. Besonders vorteilhaft wird als molekularen Sauerstoff enthaltendes Gas Luft verwendet.

Die erfindungsgemäßen Katalysatoren können alleine oder in Kombination mit anderen, unterschiedlich aktiven Katalysatoren, beispielsweise Katalysatoren des Standes der Technik auf Vanadiumoxid/Anatas-Basis, eingesetzt werden, wobei die unterschiedlichen Katalysatoren im Allgemeinen in separaten Katalysatorschüttungen, die in einem oder mehreren Katalysatorfestbetten angeordnet sein können, im Reaktor angeordnet werden.

Bei einer bevorzugten Ausführungsform des Verfahrens wird der aromatische Kohlenwasserstoff zunächst an einer Schüttung des erfindungsgemäßen Katalysators unter Teilumsatz zu einem Reaktionsgemisch umgesetzt. Das erhaltene Reaktionsgemisch oder eine Fraktion davon kann man dann mit wenigstens einem weiteren Katalysator in Kontakt bringen, dessen katalytisch aktive Masse Vanadiumpentoxid und Anatas enthält.

Bei einer bevorzugten Ausführungsform eines Verfahrens zur Herstellung von Phthalsäureanhydrid aus o-Xylol leitet man einen gasförmigen Strom, der o-Xylol und molekularen Sauerstoff enthält, nacheinander über ein Bett eines stromaufwärts gelegenen Katalysators und ein Bett eines stromabwärts gelegenen Katalysators, wobei das Bett des stromaufwärts gelegenen Katalysators einen Katalysator enthält, dessen katalytisch aktive Masse ein Metallmischoxid der allgemeinen Formel I umfasst, und das Bett des stromabwärts gelegenen Katalysators wenigstens einen Katalysator enthält, dessen katalytisch aktive Masse Titandioxid und Vanadiumpentoxid umfasst.

Alternativ werden die erfindungsgemäßen Katalysatoren für die Ammoxidation von aromatischen Kohlenwasserstoffen zu Nitrilen verwendet. Hierzu bringt man ein gasförmiges Gemisch, das den aromatischen Kohlenwasserstoff, Ammoniak und molekularen Sauerstoff enthält, bei erhöhter Temperatur, z. B. 300 bis 500 °C, vorzugsweise 420 bis 480 °C, mit dem Katalysator in Kontakt. Die Umsetzung kann in einem Festbettreaktor mit ortfest angeordnetem Katalysatorfestbett oder einem Wirbelschichtreaktor erfolgen. Die stark exotherme Ammonoxidation wird technisch üblicherweise in Wirbelbettreaktoren durchführt.

Die Sauerstoffkonzentration in dem Reaktionsgemisch kann in weiten Grenzen variieren. Vorzugsweise verwendet man einen Sauerstoffüberschuss, etwa das 1,5-fache der theoretisch erforderlichen Menge. An Ammoniak setzt man zweckmäßig einen Überschuss ein, vorteilhaft das 1,2- bis 20-fache der theoretisch erforderlichen Menge.

Die Konzentration des aromatischen Kohlenwasserstoffs in dem Gemisch der Reaktionsteilnehmer beträgt vorzugsweise 1,8 bis 5,0 Volumenprozent, bezogen auf die gesamte gasförmige Reaktionsmischung. Man kann auch eine Verdünnung mit Inertgasen vornehmen, indem man z.B. Luft als Sauerstoff lieferndes Gas verwendet oder indem man das Gasgemisch mit Inertgasen, z.B. mit Stickstoff verdünnt.

Die Verweilzeit des Gasgemisches am Katalysator kann in weiten Grenzen, z.B. zwischen 0,1 und 25 Sekunden varrieren, vorzugsweise beträgt sie etwa 0,5 bis 5 Sekunden.

Aus den Reaktionsgasen der Partialoxidation bzw. Ammoxidation kann man die gewünschten Reaktionsprodukte in an sich bekannter Weise durch Kondensation, gegebenenfalls durch Einsprühen von Wasser, abscheiden.

Die Erfindung wird durch die folgenden Beispiele näher veranschaulicht.

### Beispiele

### Herstellungsbeispiel 1: Vollkatalysator der Zusammensetzung Mo₃Fe₂Ag_{0,5}Oₓ

561 ml Eisen(II)-nitratlösung (Riedel-de-Hähn) der Konzentration 0,24 mol/l, 357 ml Ammoniumheptamolybdatlösung (Merck) der Konzentration 0,56 mol/l und 82 ml Silbernitratlösung (ABCR) der Konzentration 0,41 mol/l wurden gemischt und bis zur Trockene eingedampft. Anschließend wurde der ockerfarbene Feststoff bei 120 °C für 16 h im Trockenschrank getrocknet. Die darauf folgende Calcination erfolgte unter Luft. Es wurde mit einer Aufheizrate von 1,5 °C/min auf 450°C aufgeheizt und die Temperatur für 300 min dort gehalten.

Das erhaltene Pulver wurde mit einem Kompaktor (Powtec RC 100 x 30) kompaktiert. Die Kornfraktion von 0,4 bis 0,7 mm wurde in der katalytischen Testung eingesetzt. Die Stöchiometrie des Katalysators wurde mittels RFA-Analyse untersucht und ergab 7,9 Gewichts-% Ag, 17,3 Gewichts-% Fe und 44,0 Gewichts-% Mo. Das erhaltene Mischmetalloxid hatte eine spezifische Oberfläche 1,9 m²/g.

Es wurde ein Pulverröntgendiagramm mit Hilfe eines Siemens Difraktometer D 5000 unter Anwendung von Cu-Kα-Strahlung (40 kV, 40 mA) aufgenommen. Das Diffraktometer war mit einem automatischen Primär- und Sekundärblendensystem sowie einem Sekundär-Monochromator und Szintillations-Detektor ausgestattet. Tabelle 1 zeigt das am erhaltenen Mischmetalloxid im 2θ-Bereich von 5 bis 70° gemessene Pulverröntgendiagramm, wiedergegeben in Gestalt der von der Wellenlänge der verwendeten Röntgenstrahlung unabhängigen Netzebenenabstände d [Angström] sowie die zugehörigen, auf den intensitätstärksten Beugungsreflex bezogenen, relativen Intensitäten Iᵣₑₗ [%] der verschieden Beugungsreflexe. Die relativen Intensitäten wurden aus den Peakhöhen der Beugungsreflexe ermittelt.

**Tabelle 1**

| d | Irel | | d | Irel | | d | Irel |
|---|---|---|---|---|---|---|---|
| [Angström] | [%] | | [Angström] | [%] | | [Angström] | [%] |
| 7,852 | 1,90 | | 2,504 | 5,40 | | 1,734 | 17,10 |
| 6,868 | 1,50 | | 2,491 | 9,10 | | 1,730 | 17,30 |
| 6,409 | 11,50 | | 2,482 | 6,20 | | 1,725 | 19,00 |
| 5,780 | 21,90 | | 2,467 | 4,00 | | 1,721 | 21,30 |
| 4,938 | 3,90 | | 2,407 | 5,40 | | 1,714 | 22,90 |
| 4,639 | 11,10 | | 2,396 | 11,10 | | 1,711 | 19,50 |
| 4,556 | 12,20 | | 2,388 | 12,30 | | 1,705 | 10,00 |
| 4,402 | 10,60 | | 2,356 | 14,20 | | 1,697 | 5,40 |
| 4,393 | 8,20 | | 2,322 | 8,80 | | 1,691 | 5,90 |
| 4,339 | 38,10 | | 2,280 | 5,40 | | 1,687 | 11,30 |
| 4,081 | 52,50 | | 2,259 | 3,70 | | 1,683 | 7,20 |
| 3,998 | 37,40 | | 2,242 | 9,50 | | 1,678 | 7,90 |
| 3,918 | 45,30 | | 2,205 | 24,40 | | 1,673 | 12,60 |
| 3,874 | 91,70 | | 2,191 | 10,60 | | 1,654 | 6,00 |
| 3,860 | 100,00 | | 2,175 | 11,30 | | 1,645 | 7,50 |
| 3,848 | 98,20 | | 2,137 | 11,50 | | 1,639 | 6,40 |
| 3,744 | 14,30 | | 2,089 | 4,40 | | 1,626 | 20,20 |
| 3,701 | 7,30 | | 2,070 | 16,00 | | 1,623 | 22,50 |
| 3,639 | 5,50 | | 2,045 | 9,00 | | 1,602 | 13,10 |
| 3,565 | 28,90 | | 2,015 | 13,10 | | 1,594 | 10,60 |
| 3,560 | 28,60 | | 2,007 | 6,70 | | 1,574 | 4,40 |
| 3,461 | 60,30 | | 2,000 | 11,20 | | 1,566 | 12,10 |
| 3,376 | 3,70 | | 1,992 | 5,50 | | 1,552 | 10,10 |
| 3,347 | 10,40 | | 1,959 | 6,70 | | 1,547 | 7,20 |
| 3,242 | 55,80 | | 1,937 | 9,30 | | 1,538 | 9,00 |
| 3,204 | 21,80 | | 1,932 | 8,40 | | 1,528 | 7,40 |
| 3,144 | 11,90 | | 1,921 | 19,00 | | 1,509 | 4,50 |
| 3,140 | 13,90 | | 1,916 | 13,50 | | 1,504 | 5,60 |
| 3,059 | 2,00 | | 1,896 | 12,50 | | 1,493 | 6,80 |
| 2,967 | 22,20 | | 1,893 | 10,90 | | 1,487 | 7,20 |
| 2,958 | 32,60 | | 1,873 | 11,70 | | 1,478 | 7,00 |
| 2,891 | 12,90 | | 1,854 | 5,60 | | 1,458 | 17,80 |
| 2,842 | 24,70 | | 1,847 | 8,10 | | 1,435 | 11,00 |
| 2,811 | 33,00 | | 1,840 | 4,30 | | 1,417 | 12,40 |
| 2,788 | 71,40 | | 1,812 | 6,10 | | 1,395 | 7,40 |
| 2,752 | 43,60 | | 1,804 | 7,00 | | 1,376 | 2,30 |
| 2,750 | 38,50 | | 1,794 | 5,60 | | 1,370 | 4,20 |
| 2,653 | 17,30 | | 1,784 | 13,10 | | 1,355 | 6,20 |
| 2,630 | 48,60 | | 1,772 | 7,40 | | 1,346 | 2,40 |
| 2,613 | 13,00 | | 1,766 | 8,00 | | | |
| 2,582 | 11,00 | | 1,763 | 7,30 | | | |
| 2,549 | 6,40 | | 1,761 | 5,60 | | | |
| 2,524 | 6,10 | | 1,740 | 21,40 | | | |

Herstellungsbeispiel 2: Schalenkatalysator mit der Aktivmasse Mo₃Fe₂Ag₄Oₓ 814 ml Eisen(II)-nitratlösung (Riedel-de-Hähn) der Konzentration 0,24 mol/l, 345 ml Ammoniumheptamolybdatlösung (Merck) der Konzentration 0,85 mol/l und 119 ml Silbernitratlösung (ABCR) der Konzentration 0,41 mol/l wurden gemischt und bis zur Trockene eingedampft. Anschließend wurde der ockerfarbene Feststoff bei 120 °C für 16 im Trockenschrank getrocknet. Die darauf folgende Calcination erfolgte unter Luft. Es wurde mit einer Aufheizrate von 1,5 °C/min auf 450°C aufgeheizt und die Temperatur für 300 min dort gehalten.

Das erhaltene Pulver wurde durch ein Sieb mit Maschenweite 0,12 mm gesiebt. Die Stöchiometrie des Pulvers wurde mittels RFA-Analyse untersucht und ergab 6,3 Gewichts-% Ag, 17,0 Gewichts-% Fe und 44,0 Gewichts-% Mo. Das erhaltene Mischmetalloxid hatte eine spezifische Oberfläche 1,6 m²/g. Das wie zuvor beschrieben aufgenommene Pulverröntgendiagramm ist in Tabelle 2 dargestellt.

**Tabelle 2**

| d | Irel | | d | Irel | | d | Irel |
|---|---|---|---|---|---|---|---|
| [Angstrom] | [%] | | [Angstrom] | [%] | | [Angstrom] | [%] |
| 7,861 | 1,10 | | 2,894 | 11,60 | | 2,176 | 11,00 |
| 6,420 | 10,50 | | 2,881 | 8,00 | | 2,138 | 11,00 |
| 6,041 | 1,50 | | 2,872 | 7,10 | | 2,118 | 2,00 |
| 5,793 | 18,00 | | 2,844 | 23,80 | | 2,092 | 4,00 |
| 4,948 | 2,90 | | 2,813 | 28,70 | | 2,070 | 14,70 |
| 4,645 | 8,20 | | 2,791 | 61,90 | | 2,047 | 8,00 |
| 4,560 | 10,00 | | 2,755 | 38,30 | | 2,028 | 5,10 |
| 4,403 | 7,80 | | 2,702 | 4,50 | | 2,017 | 12,40 |
| 4,349 | 31,30 | | 2,656 | 16,70 | | 2,009 | 9,90 |
| 4,269 | 2,00 | | 2,632 | 42,40 | | 2,001 | 10,60 |
| 4,086 | 46,50 | | 2,629 | 41,70 | | 1,959 | 7,50 |
| 4,002 | 31,70 | | 2,583 | 9,20 | | 1,938 | 10,60 |
| 3,921 | 41,80 | | 2,551 | 6,00 | | 1,932 | 8,10 |
| 3,876 | 94,40 | | 2,527 | 6,50 | | 1,921 | 17,70 |
| 3,867 | 100,00 | | 2,518 | 5,50 | | 1,897 | 13,90 |
| 3,850 | 86,40 | | 2,503 | 6,00 | | 1,893 | 12,00 |
| 3,746 | 15,20 | | 2,492 | 7,60 | | 1,873 | 12,30 |
| 3,721 | 6,80 | | 2,469 | 3,70 | | 1,852 | 5,80 |
| 3,711 | 7,40 | | 2,408 | 5,30 | | 1,847 | 7,30 |
| 3,643 | 5,90 | | 2,397 | 12,10 | | 1,843 | 6,90 |
| 3,572 | 27,90 | | 2,389 | 12,90 | | 1,812 | 6,30 |
| 3,465 | 57,60 | | 2,357 | 12,60 | | 1,807 | 8,20 |
| 3,371 | 4,80 | | 2,323 | 6,90 | | 1,802 | 7,10 |
| 3,350 | 10,20 | | 2,282 | 5,90 | | 1,796 | 6,60 |
| 3,244 | 46,40 | | 2,258 | 4,20 | | 1,785 | 11,70 |
| 3,205 | 24,20 | | 2,249 | 5,30 | | 1,771 | 9,20 |
| 3,143 | 14,00 | | 2,243 | 9,60 | | 1,763 | 8,70 |
| 3,139 | 13,20 | | 2,204 | 24,50 | | 1,741 | 20,70 |
| 3,071 | 2,80 | | 2,201 | 17,80 | | 1,732 | 16,80 |
| 2,962 | 28,20 | | 2,189 | 9,10 | | 1,721 | 23,30 |
| 1,714 | 19,40 | | 1,509 | 6,10 | | 1,344 | 4,40 |
| 1,696 | 7,00 | | 1,505 | 5,90 | | 1,340 | 5,70 |
| 1,687 | 11,70 | | 1,493 | 8,70 | | 1,337 | 6,30 |
| 1,674 | 13,10 | | 1,489 | 9,00 | | 1,320 | 6,00 |
| 1,653 | 6,10 | | 1,484 | 7,10 | | 1,313 | 3,90 |
| 1,645 | 8,70 | | 1,478 | 8,10 | | 1,307 | 4,50 |
| 1,640 | 8,20 | | 1,458 | 17,10 | | 1,295 | 4,10 |
| 1,627 | 20,60 | | 1,448 | 6,90 | | 1,289 | 6,30 |
| 1,623 | 23,90 | | 1,438 | 7,50 | | 1,286 | 4,50 |
| 1,609 | 8,00 | | 1,435 | 12,50 | | 1,282 | 3,20 |
| 1,604 | 13,00 | | 1,417 | 15,60 | | 1,280 | 2,90 |
| 1,594 | 11,30 | | 1,408 | 5,10 | | 1,267 | 3,00 |
| 1,590 | 10,70 | | 1,400 | 5,20 | | 1,265 | 3,20 |
| 1,573 | 6,60 | | 1,396 | 8,40 | | 1,261 | 5,60 |
| 1,566 | 13,30 | | 1,388 | 4,10 | | 1,250 | 1,40 |
| 1,557 | 7,70 | | 1,370 | 5,80 | | 1,238 | 1,60 |
| 1,552 | 11,10 | | 1,361 | 7,90 | | 1,225 | 1,80 |
| 1,540 | 9,80 | | 1,356 | 8,30 | | 1,222 | 1,90 |
| 1,528 | 7,80 | | 1,347 | 4,40 | | 1,213 | 1,50 |

Das erhaltene Pulver wurde wie folgt auf Magnesiumsilikat-Kugeln aufgebracht: 300,08 g Steatitkugeln (Durchmesser 3,5 - 4,5 mm) wurden in einer Dragiertrommel bei 20°C während 25 Minuten mit 40,08 g des erhaltenen Mo₃Fe₂Ag₄Oₓ-Pulvers unter Zusatz von 8 ml eines 30 Gew.-% Glycerin und 70 Gew.-% Wasser enthaltenden Gemisches beschichtet und anschließend getrocknet bei 100 °C für 1,5 h. Das Gewicht der so aufgetragenen katalytisch aktiven Masse betrug 10,44 Gew.-%, bezogen auf das Gesamtgewicht des fertigen Katalysators (bestimmt an einer Probe des Katalysators, die einer Wärmebehandlung bei 450 °C für 1,5 h unterzogen worden war). Die darauf folgende Calcination erfolgte unter Luft. Es wurde mit einer Aufheizrate von 1,5 °C/min auf 450°C aufgeheizt und die Temperatur für 300 min dort gehalten.

### Herstellungsbeispiel 3: Undotierter Vergleichskatalysator

23968 ml Eisen(II)-nitratlösung (Riedel-de-Hähn) der Konzentration 0,480 mol/l, 15188 ml Ammoniumheptamolybdatlösung (Merck) der Konzentration 1,132 mol/l wurden gemischt und bis zur Trockene eingedampft. Anschließend wurde der ockerfarbene Feststoff bei 120 °C für 16 h im Trockenschrank getrocknet. Die darauf folgende Calcination erfolgte unter Luft. Es wurde mit einer Aufheizrate von 1,5 °C/min auf 450°C aufgeheizt und die Temperatur für 300 min dort gehalten.

Das erhaltene Pulver wurde mit einem Kompaktor (Powtec RC 100 x 30) kompaktiert. Die Kornfraktion von 0,4 bis 0,7 mm wurde in der katalytischen Testung eingesetzt.

### Oxidationsreaktionen

### Beispiel 1: Herstellung von m-Toluylaldehyd

In ein 10 cm langes elektrisch beheizbares Eisenrohr mit einer lichten Weite von 6 mm wurden von unten nach oben 1 ml Korund (Kornfraktion: 0,16 - 0,35 mm), 0,5 ml Katalysator gemäß Herstellungsbeispiel 1 (Kornfraktion: 0,4 - 0,7 mm) und 1 ml Korund (Kornfraktion: 0,16 - 0,35 mm) eingefüllt. Dann wurde das Rohr unter einem Stickstoffstrom von oben nach unten (4,5 NI/h) auf 325°C erhitzt. Nach Erreichen der Temperatur wurde für 30 min 4,5 NI/h Gas bestehend aus 10,5 Vol-% Wasser und 88,5 % Luft dosiert und danach die Reaktionsbedingungen entsprechend Tabelle 3 eingestellt. Jede Phase wurde für 12 h eingestellt.

Das gasförmige Reaktionsgemisch wurde wie Gaschromatograph gekoppelt mit Massenspektrometer (GC-MS)analysiert. Es wurden die m-Toluylaldehyd-, CO- und CO₂-Selektivität berechnet.

**Tabelle 3**

| Phase | Temp. [°C] | GHSV [1/h] | Druck [bar] | m-Xylol [Vol.-%] | O₂ [Vol.-%] | H₂O [Vol.-%] | N₂ [Vol.-%] |
|---|---|---|---|---|---|---|---|
| 1 | 325 | 9000 | 0,5 | 1 | 10,5 | 10 | 78,5 |
| 2 | 375 | 9000 | 0,5 | 1 | 10,5 | 10 | 78,5 |
| 3 | 400 | 9000 | 0,5 | 1 | 10,5 | 10 | 78,5 |
| 4 | 400 | 9000 | 0,5 | 0,5 | 5,5 | 10 | 84 |
| 5 | 450 | 9000 | 0,5 | 0,5 | 5,5 | 10 | 84 |
| 6 | 450 | 9000 | 0,5 | 0,5 | 2,5 | 10 | 87 |

Die erhaltenen Ergebnisse sind in Tabelle 4 zusammengefasst.

**Tabelle 4**

| Phase | C-Bilanz | Umsatz m-Xylol | Selektivität m-Toluyl-aldehyd | Selektivität CO | Selektivität CO₂ |
|---|---|---|---|---|---|
| 1 | 0,99 | 2,1 | 71,0 | 0,0 | 0,0 |
| 2 | 1,02 | 12,8 | 97,5 | 0,0 | 0,0 |
| 3 | 0,98 | 14,3 | 83,9 | 0,0 | 0,0 |
| 4 | 1,01 | 32,9 | 93,0 | 0,0 | 0,0 |
| 5 | 0,96 | 31,5 | 68,6 | 0,0 | 0,0 |
| 6 | 1,04 | 37,5 | 94,7 | 0,0 | 0,0 |

(C-Bilanz: Summe der Kohlenstoffstoffmenge von allen gemessenen kohlenstoffhaltigen Produkten geteilt durch Kohlenstoffstoffmenge im Eingangsgasstrom)

### Vergleichsbeispiel:

Beispiel 1 wurde wiederholt, wobei der Katalysator gemäß Herstellungsbeispiel 3 verwendet wurde.

Die erhaltenen Ergebnisse sind in Tabelle 5 zusammengefasst.

**Tabelle 5**

| Phase | C-Bilanz | Umsatz m-Xylol | Selektivität m-Toluyl-aldehyd | Selektivität CO | Selektivität CO₂ |
|---|---|---|---|---|---|
| 1 | 0,93 | 12 | 15,1 | 0,0 | 0,0 |
| 2 | 0,95 | 100 | 4,9 | 0,0 | 90,8 |
| 3 | 0,92 | 100 | 0,8 | 0,0 | 90,7 |
| 4 | 0,93 | 100 | 4,2 | 0,0 | 89,7 |
| 5 | 1,01 | 100 | 0,0 | 0,0 | 100,6 |
| 6 | 0,93 | 48 | 23,1 | 0,0 | 61,7 |

## Patentansprüche

1. Verfahren zur Partialoxidation oder Ammoxidation von aromatischen Kohlenwasserstoffen in der Gasphase unter Verwendung eines Katalysators, dessen katalytisch aktive Masse ein Metallmischoxid der allgemeinen Formel I enthält
Mo₃Fe_{b}XₓO_{z}, I
worin
X für wenigstens ein unter Ag, Te, K, Bi, Au, Ce, Co, Cs, Cu, Eu, Mn, Rb, Sb, Se, Si, V, W ausgewähltes Metall steht,
b einen Wert von 1,6 bis 2,5 hat,
x einen Wert von 0,01 bis 3,0 hat
z eine Zahl, die sich durch die Wertigkeit und Häufigkeit der von Sauerstoff verschiedenen Elemente in der Formel I bestimmt, bedeutet.

2. Verfahren nach Anspruch 1, wobei
b einen Wert von 1,8 bis 2,3 hat.

3. Verfahren nach Anspruch 1 oder 2, wobei
x einen Wert von 0,05 bis 1 hat.

4. Verfahren nach einem der vorhergehenden Ansprüche, wobei X für Ag steht.

5. Verfahren nach einem der vorhergehenden Ansprüche, wobei der Kohlenwasserstoff unter ein- oder mehrfach alkylierten Aromaten und kondensierten Aromaten ausgewählt ist.

6. Verfahren nach Anspruch 5, wobei der Kohlenwasserstoff unter Toluol, o-Xylol, m-Xylol, p-Xylol und Naphthalin ausgewählt ist.

7. Verfahren nach Anspruch 5, zur Herstellung von m-Toluylaldehyd aus m-Xylol.

8. Verfahren nach Anspruch 5, zur Herstellung von o-Toluylaldehyd und/oder Phthalsäureanhydrid aus o-Xylol und/oder Naphthalin.

9. Verfahren nach Anspruch 8, wobei man einen gasförmigen Strom, der o-Xylol und molekularen Sauerstoff enthält, nacheinander über ein Bett eines stromaufwärts gelegenen Katalysators und ein Bett eines stromabwärts gelegenen Katalysators leitet, wobei das Bett des stromaufwärts gelegenen Katalysators einen Katalysator enthält, dessen katalytisch aktive Masse ein Metallmischoxid der allgemeinen Formel I umfasst, und das Bett des stromabwärts gelegenen Katalysators wenigstens einen Katalysator enthält, dessen katalytisch aktive Masse Titandioxid und Vanadiumpentoxid umfasst.

10. Gasphasenoxidations-Katalysator, umfassend eine katalytisch aktive Masse, die ein Metallmischoxid der allgemeinen Formel I umfasst
Mo₃Fe_{b}XₓO_{z}, I
worin
X für wenigstens ein unter Ag, Te, K, Bi, Au, Ce, Co, Cs, Cu, Eu, Mn, Rb, Sb, Se, Si, V, W ausgewähltes Metall steht,
b einen Wert von 1,6 bis 2,5 hat,
x einen Wert von 0,01 bis 3,0 hat
z eine Zahl, die sich durch die Wertigkeit und Häufigkeit der von Sauerstoff verschiedenen Elemente in der Formel I bestimmt, bedeutet.
